# FASCICULE DE BREVET EUROPEEN

(11) **EP 1 778 290 B1**
(45) Date de publication et mention de la délivrance du brevet: **07.06.2017**
(21) Numéro de dépôt: 05753823.3
(22) Date de dépôt: 07.04.2005
(51) Int. Cl.: A61K 47/44, A61K 38/21, A61K 9/50

(54) **UTILISATION DU DIPALMITOSTEARATE DE GLYCEROL POUR AMELIORER LA BIODISPONIBILITE DE PRINCIPES ACTIFS PROTEIQUES EN FORMULATIONS INJECTABLES SOUS-CUTANEES OU INTRAMUSCULAIRES**
VERWENDUNG VON GLYCEROLDIPALMITOSTEARAT ZUR VERBESSERUNG DER BIOVERFÜGBARKEIT VON PROTEINWIRKSTOFFEN IN SUBKUTANEN ODER INTRAMUSKULÄREN INJEKTIONSFORMULIERUNGEN
USE OF GLYCEROL DIPALMITOSTEARATE FOR IMPROVING THE BIOAVAILABILITY OF PROTEIN ACTIVE INGREDIENTS IN SUBCUTANEOUS OR INTRAMUSCULAR INJECTABLE FORMULATIONS

(30) Priorité: 07.04.2004 FR 0403639
(43) Date de publication de la demande: 02.05.2007
(73) Titulaire: Ethypharm, 92210 Saint-Cloud (FR)
(72) Inventeur: RICHARD, Jöel Parco di Villa Grazioli, 00046 Grottaferrata (IT); DESCHAMPS, Frantz, F-54000 Nancy (FR); DE CONTI, Anne-Marie, F-94100 Saint-Maur-Des-Fossés (FR); THOMAS, Olivier, F-49100 Angers (FR); AUBRETON, Richard, F-49100 Angers (FR)
(74) Mandataire: Regimbeau
(86) Numéro de dépôt international: PCT/FR2005/000849
(87) Numéro de publication internationale: WO 2005/099767

(56) Documents cités:
- WO-A-00/03660
- WO-A-00/38652
- WO-A-01/49268
- WO-A-01/89481
- WO-A-92/05771
- WO-A-94/08623
- WO-A-97/34584
- WO-A-03/063841
- US-A- 5 520 927
- US-A- 5 925 739
- US-A1- 2003 007 930
- PONGJANYAKUL THANED ET AL: "Melted glyceryl palmitostearate (GPS) pellets for protein delivery." INTERNATIONAL JOURNAL OF PHARMACEUTICS (KIDLINGTON), vol. 271, no. 1-2, 1 mars 2004 (2004-03-01), pages 53-62, XP002343538 ISSN: 0378-5173
- REITHMEIER HELMUT ET AL: "Lipid microparticles as a parenteral controlled release device for peptides" JOURNAL OF CONTROLLED RELEASE, vol. 73, no. 2-3, 15 juin 2001 (2001-06-15), pages 339-350, XP002343539 ISSN: 0168-3659
- GATTEFOSSÉ: 'Biogapress Vegetal BM297ATO Glyceryl dipalmitostearate E471/GRAS', [en ligne] 2010, pages 1 - 1, XP055015562 Extrait de l'Internet: <URL:http://www.gattefosse.com/en/applicati ons/biogapress-vegetal-bm297-ato.html> [extrait le 2012-01-02]
- GATTEFOSSÉ: 'Precirol ATO 5 Glycerol distearate (type I) EP', [en ligne] 2010, pages 1 - 1, XP055015563 Extrait de l'Internet: <URL:http://www.gattefosse.com/en/applicati ons/precirol-ato5.html> [extrait le 2012-01-02]
- Gattefosse: "Precirol ATO 5", , 1 January 2010 (2010-01-01), XP055285325, Retrieved from the Internet: URL:http://www.gattefosse.com/fr/applicati ons/precirol-ato5.html [retrieved on 2016-07-01]

## Description

La présente invention concerne une méthode pour augmenter la biodisponibilité de principes actifs protéiques en formulations injectables sous-cutanées ou intramusculaires, ledit principe actif étant l'interféron α-2b.

Les avancées récentes dans le domaine des biotechnologies, et plus particulièrement du génie génétique, ont conduit au développement de nombreux principes actifs protéiques, définis comme les peptides et les protéines, notamment pour une utilisation dans le traitement de pathologies lourdes (cancers, anémies, sclérose multiple, hépatites, etc.). Cependant, pour ces principes actifs macromoléculaires qui sont fragiles et peu biodisponibles, le développement de formulations stables, efficaces et bien tolérées par le patient reste un véritable challenge. En particulier, à cause de leur faible stabilité dans le tractus gastro-intestinal (dégradation à faible pH et protéolyse) et de leur faible perméabilité à travers la barrière intestinale, les principes actifs protéiques ne peuvent être administrés par la voie orale. Dans ce domaine, bien qu'une amélioration de la perméabilité des peptides et protéines à travers la membrane épithéliale soit envisageable, la biodisponibilité orale reste néanmoins inférieure à quelques pour cent, ce qui empêche le développement de formes orales de ces principes actifs. L'administration par voie injectable ou parentérale reste donc dans ce contexte la voie privilégiée d'administration des principes actifs protéiques. Ainsi, le développement de nouvelles formes pharmaceutiques injectables ou de dispositifs d'injection est vraisemblablement plus à même de remporter un succès thérapeutique et commercial que l'utilisation d'une nouvelle voie d'administration, à condition toutefois que les injections assurent la sécurité et l'efficacité désirées. Une revue sur l'état de l'art des nouvelles formes pharmaceutiques des produits issus des biotechnologies a été publiée récemment par G. Orive et al. ("Drug delivery in biotechnology: present and future"; Current Opinion in Biotechnology 14, 2003, 659-664).

De nombreuses formulations de peptides et protéines injectables par voie intraveineuse, intramusculaire ou sous-cutanée sont déjà commercialisées ou en cours de développement. Une revue des formulations des principes actifs protéiques disponibles commercialement ou en cours de développement a été publiée récemment par J.L. Cleland et al. ("Emerging protein delivery methods"; Current Opinion in Biotechnology 12, 2001, 212-219). Comme le montrent ces auteurs, ces formulations nécessitent encore des améliorations importantes, liées à plusieurs problèmes qui restent non résolus. Tout d'abord, la bonne observance du traitement est une préoccupation majeure dans le traitement des populations âgées ou des jeunes enfants, où les injections parentérales répétées sont très mal acceptées. De plus, des effets secondaires fréquents, dus directement à l'injection et au pic de concentration plasmatique, tels que le syndrome pseudo-grippal, l'anorexie, l'amaigrissement, les troubles digestifs, etc. sont également observés. Enfin, les concentrations systémiques élevées nécessaires pour que ces principes actifs protéiques produisent l'effet souhaité sur les tissus ou organes visés, sont très souvent responsables d'une toxicité systémique (hématologique...) néfaste et préjudiciable pour le patient ; ces concentrations élevées, nécessaires pour se situer dans la fenêtre d'efficacité thérapeutique, sont obtenues par injection fréquente de doses élevées. Les deux premiers problèmes ont trouvé des solutions partielles dans les systèmes de délivrance prolongée de principes actifs protéiques à base de polymères (implants, microsphères, gels), bien que ces systèmes n'aient pas résolu le problème de perte d'activité (dénaturation) et d'agrégation et/ou précipitation, lié aux procédés et aux excipients de formulation utilisés (voir par exemple l'article de M. van de Weert et al.: "Protein instability in poly(lactic-co-glycolic acid) microparticles"; Pharm. Res. 17, 2000, 1159-1167). En revanche, le troisième point, qui relève de l'exposition du patient au principe actif protéique, reste aujourd'hui non ré solu, et conduit dans de nombreux cas à l'échappement de ce traitement par le patient. Ce problème est lié à la faible biodisponibilité des principes actifs protéiques, et plus particulièrement des protéines administrées par voie sous-cutanée ou intramusculaire, qui conduit à l'utilisation de doses thérapeutiques élevées. Après administration sous-cutanée (s.c.), la biodisponibilité absolue (versus une administration intraveineuse à la même dose) est couramment comprise entre 12 et 70% pour les protéines de masse molaire supérieure ou égale à 20 000 g/mole (érythropoïétine, interféron-beta, interféron-gamma, etc.); elle peut être légèrement supérieure et atteindre environ 80% pour les protéines de masse molaire plus faible. De même, la biodisponibilité absolue de certaines cytokines telles que l'interféron-beta, après administration intramusculaire (i.m.) est très faible (inférieure ou égale à 10%) (voir par exemple l'article de V. Bocci: "Physicochemical and biologie properties of interferons and their potential uses in drug delivery systems"; Critical Reviews in Therapeutic Carrier Systems 9 (2), 1992, 91-133). Ce problème de biodisponibilité après administration sous-cutanée persiste également pour les nouvelles formes de bioconjugués protéiques à temps de circulation systémique prolongé, telles que les protéines PEGylées (*i.e.* sur lesquelles on a fixé de façon covalente des groupements polymères hydrosolubles de type poly(éthylèneglycol) ou (PEG)), ou les protéines glycosylées (*i.e.* sur lesquelles on a fixé de façon covalente des groupements polymères hydrosolubles de type mono- ou polysaccharide), comme il a été montré en 2003 dans la revue de P. Caliceti et al. ("Pharmacokinetic and biodistribution properties of poly(ethyleneglycol)-protein conjugates"; Advanced Drug Delivery Reviews 55, 2003, 1261-1277). WO01/49268 divulgue une formulation matricielle à base d'un polymère hydrophile (PEG) et optionnellement d'un résidu phospholipidique pour augmenter la biodisponibilité d'un principe actif, notamment l'interféron alpha-2b. L'origine exacte de la faible biodisponibilité absolue après injection s.c. ou i.m. n'est pas élucidée, mais elle est attribuée par la littérature à une possible dégradation (protéolyse) ou métabolisation du principe actif protéique au site d'injection ou lors du transport lymphatique vers le plasma. La dégradation des cytokines par les protéases après administration s.c. réduit ainsi leur biodisponibilité de plus de 30% (voir par exemple l'article de B. Rouveix: "Clinical pharmacology of cytokines"; Eur. Cytokine Network 8 (3), 1997, 291-293). Une revue de l'état des connaissances sur l'absorption des protéines et les mécanismes de transport vers la circulation sanguine après administration par injection s.c. a été publiée récemment par C.J.H. Porter et al. (« Lymphatic transport of proteins after subcutaneous administration » ; J. Pharm. Sci 89 (3), 2000, 297-310).

Enfin, il faut remarquer que si la biodisponibilité des proteins injectées par voie s.c. ou i.m. est faible (<20%), cela génère alors une perte importante de la protéine dont une petite partie seulement sera thérapeutiquement utile, ce qui conduit à une augmentation significative du coût de production du médicament pour la société pharmaceutique productrice (J.L. Cleland et *al.* Déjà cité).

Dans ce contexte, le demandeur a découvert que la formulation de l'interféron α-2b avec des composés lipidiques utilisés comme agents de formulation permet, de façon inattendue, d'augmenter la biodisponibilité absolue de ces principes actifs après administration par voie injectable sous-cutanée ou intramusculaire. Cette augmentation de biodisponibilité permet de répondre aussi bien au problème de dose thérapeutique et de toxicité associé, qu'au problème économique posé par la production du médicament contenant ces principes actifs.

Aussi, l'objet de la présente invention est-il de proposer une méthode pour améliorer la biodisponibilité de l'interféron α-2b en formulations injectables sous-cutanées ou intramusculaires.

Selon la présente invention, il a été mis en évidence que l'utilisation du distéarate de glycérol comme agent de formulation permettait d'augmenter la biodisponibilité de l'interféron α-2b en formulations injectables sous-cutanées ou intramusculaires.

L'objet de la présente invention concerne par conséquent l'utilisation du distéarate de glycérol comme agent de formulation pour augmenter la biodisponibilité de l'interféron α-2b dans des formulations injectables sous-cutanées ou intramusculaires.

Les lipides sont utilisés sous forme liquide ou solide à la température ambiante. Dans un mode avantageux de réalisation, ils sont utilisés sous forme solide.

Selon un mode très avantageux de réalisation de l'invention, le distéarate de glycérole, est du Précirol ATO® 5 (distéarate de glycérol atomisé de type I selon la nomenclature de la Pharmacopée Européenne (3ème édition)).

Selon l'invention, le principe actif protéique est l'interféron α-2b.

Les principes actifs protéiques utilisés selon l'invention sont préférentiellement contenus dans des particules de taille comprise entre 0,1 et 100 µm, encore plus avantageusement comprise entre 1 et 25 µm. Ils sont obtenus par tout procédé connu de l'homme du métier de façon non limitative : lyophilisation, nébulisation, nébulisation-congélation, cristallisation, précipitation, broyage, procédé utilisant un fluide à pression supercritique.

La teneur en protéines des particules de principe actif protéique est comprise entre 0,01 et 100%, et ajustée selon les connaissances de l'homme du métier pour obtenir la dose thérapeutique souhaitée. Ces particules de principe actif protéique peuvent contenir également des excipients et additifs couramment utilisés, tels que par exemple des tensioactifs, des polymères, des sucres, des polysaccharides, des sels, des tampons, et d'autres protéines.

Les procédés qui peuvent être mis en oeuvre pour obtenir des systèmes contenant un principe actif protéique formulé avec un agent lipidique sont nombreux et classiquement connus de l'homme du métier. Ils sont par exemple basés sur des techniques d'émulsification, dispersion, émulsion-extraction de solvant, émulsion à chaud (*hot-melt emulsion*)-refroidissement, émulsion double, émulsion de type (solide-dans-huile)-dans-eau, précipitation par basculement de solvant, coacervation (séparation de phase), nébulisation pouvant être suivie d'un refroidissement, perlage (*prilling*), enrobage en lit fluidisé, encapsulation utilisant un fluide à pression supercritique, extrusion, extrusion-sphéronisation, thermo-formage ou compression (pour les implants). Un procédé d'encapsulation utilisant un fluide à pression supercritique comme solvant ou non-solvant du ou des lipides de la formulation est préférentiellement mis en oeuvre. Dans ce cas, l'agent lipidique utilisé est de préférence soluble dans le fluide supercritique. Le fluide supercritique est préférentiellement constitué de dioxyde de carbone (CO₂), seul ou en présence de co-solvants.

Les systèmes formulés obtenus ont des structures de type nanocapsule, microcapsule (structure coeur protéique-écorce lipidique), ou nanosphère, microsphère, implants cylindriques ou discoïdes (structure matricielle lipidique contenant le principe actif protéique dispersé). Ils ont préférentiellement une structure matricielle.

Les systèmes formulés sont dépourvus de tout polymère de type PLA/PLGA ou de type polymères acryliques, cellulosiques ou vinyliques.

Leur teneur en particules de principe actif protéique (taux de charge) est comprise entre 0,1 et 95%, avantageusement entre 0,5 et 25%, encore plus avantageusement entre 1 et 10%.

Les systèmes formulés contenant le principe actif protéique ainsi obtenu ont une taille moyenne comprise entre 1 et 500 µm.

Pour les systèmes formulés particulaires lipidiques, le diamètre moyen est préférentiellement compris entre 10 et 100 µm; pour les implants lipidiques, le diamètre moyen est préférentiellement compris entre 10 et 300 µm.

Préalablement à l'injection, les systèmes particulaires sont redispersés dans un véhicule liquide pour injection sous-cutanée ou intramusculaires. De façon non limitative, il s'agit de solutions aqueuses, et préférentiellement de solutions aqueuses isotoniques, tamponnées, de viscosité définie et ajustée par des polymères hydrosolubles et/ou tensioactifs, et contenant des tensioactifs permettant d'obtenir une dispersion homogène et stable des particules. La composition de ces véhicules liquides pour injection est ajustée par l'homme du métier pour obtenir une dispersion injectable en sous-cutané ou en intramusculaire.

La méthode selon l'invention conduit à une augmentation de la biodisponibilité du principe actif protéique et permet d'utiliser des quantités moindres de principes actifs, évitant ainsi l'arrêt du traitement (échappement) lié à la toxicité élevée desdits principes actifs protéiques.

Les exemples qui suivent, les tableaux et les figures illustrent l'invention.

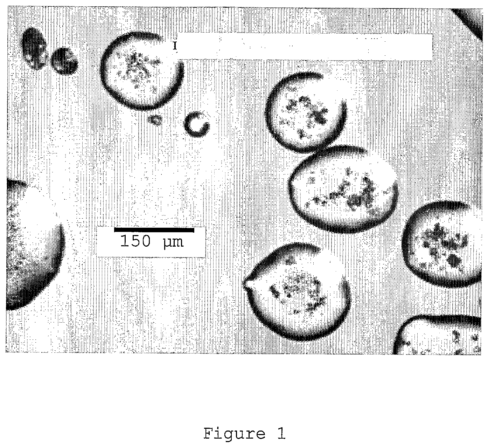

La **figure 1** est une photographie au microscope optique des microparticules M1 après fusion du lipide telles que décrites dans l'exemple 1.

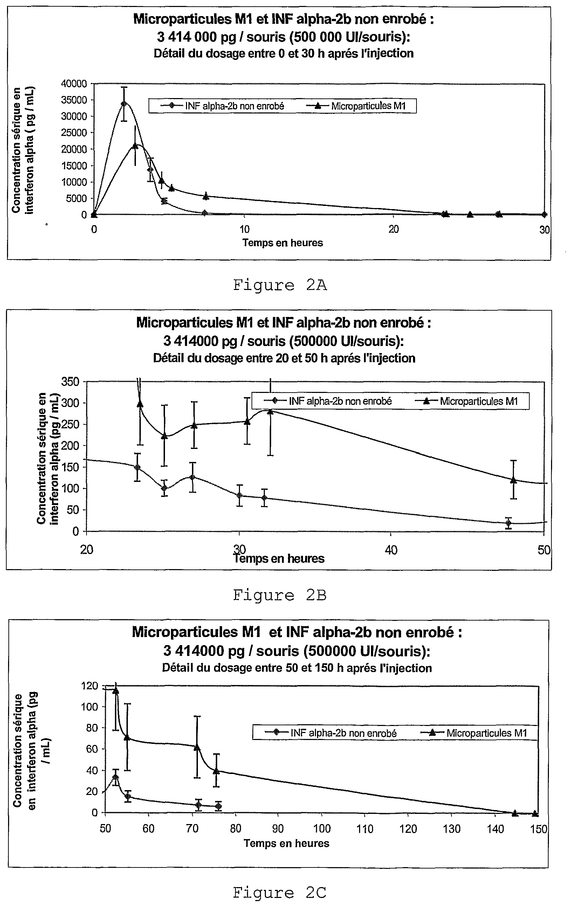

La **figure 2** représente la concentration sérique en interféron α chez la souris pour une dose d'interféron α-2b de 500 000 UI/souris: (A) 0 à 30 h après l'injection de INFα-2b non enrobé ou de microparticules M1 préparées selon l'exemple 1, (B) 20 à 50 h après l'injection de INFα-2b on enrobé ou de microparticules M1 préparées selon l'exemple 1, (C) 50 à 150 h après l'injection de INFα-2b non enrobé ou de microparticules M1 préparées selon l'exemple 1.

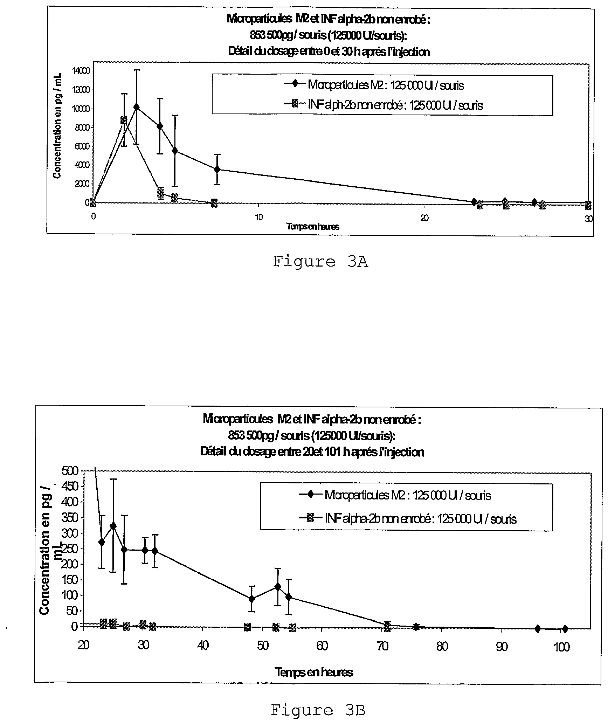

La **figure 3** représente la concentration sérique en interféron α chez la souris pour une dose de 125 000 UI/souris: (A) 0 à 30 h après l'injection de INFα-2b non enrobé ou de microparticules M2 préparées selon l'exemple 2 et suspendues dans le milieu de l'exemple 4, (B) 20 à 101 h après l'injection de INFα-2b non enrobé ou de microparticules M2 préparées selon l'exemple 2 et suspendues dans le milieu de l'exemple 4.

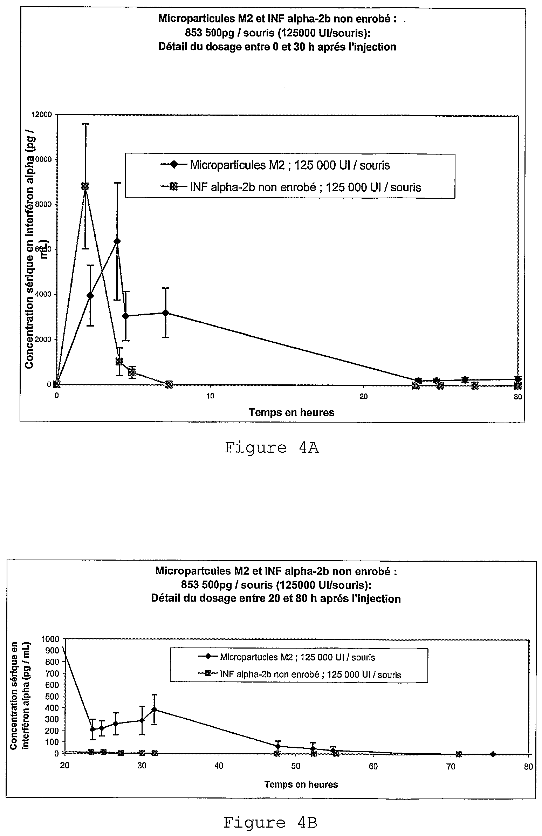

La **figure 4** représente illustre la concentration sérique en interféron α chez la souris pour une dose d'interféron α-2b de 125 000 UI/souris: (A) 0 à 30 h après l'injection de INFα-2b non enrobé ou de microparticules M2 préparées selon l'exemple 2 et suspendues dans le milieu selon l'exemple 5, (B) 20 à 80 h après l'injection de INFα-2b non enrobé ou de microparticules M2 préparées selon l'exemple 2 et suspendues dans le milieu selon l'exemple 5.

### Exemple 1: Préparation de microparticules lipidiques M1 contenant l'interféron α-2b.

Les microparticules sont préparées à partir d'un lyophilisat d'interféron α-2b (INF^{®}, Gautier Cassara, Argentine) et d'un mélange de glycérides (Précirol^{®} ATO 5, Gattefossé, France) solide à température ambiante. Le point de fusion du Précirol^{®} ATO 5 est mesuré par analyse enthalpique différentielle à balayage, et trouvé égal à 56,2°C. Préalablement à leur enrobage par le Précirol^{®} ATO 5, les particules de lyophilisat protéique INF^{®} contenant l'interféron α-2b (IFNα-2b) sont broyées à l'aide d'un mortier, puis tamisées à l'aide d'un tamis de 25 µm. On utilise la fraction tamisée inférieure à 25 µm pour la préparation des microparticules. Le diamètre moyen des particules de lyophilisat protéique broyées et tamisées, déterminé par granulométrie laser, est d'environ 15 µm. La teneur en IFNα-2b des particules protéiques à enrober est mesurée à l'aide d'un kit de dosage immuno-enzymatique (ELISA) spécifique de l'IFNα-2b humain (R&D Systems). Elle est mesurée et trouvée égale à 2,866 x 10⁶ pg d'IFNα-2b par mg de particules à enrober.

On introduit 157,45 mg de particules de lyophilisat protéique à enrober et 3 g de Précirol^{®} ATO 5 dans un réacteur inox d'un volume interne de IL. Le réacteur a une pression maximale d'utilisation de 30 MPa et est muni d'une double enveloppe permettant une régulation de température par circulation de fluide caloporteur. Le couvercle du réacteur est équipé d'un dispositif d'agitation pendulaire à entraînement magnétique. Après introduction des particules à enrober et du Précirol^{®} ATO 5, on ferme le réacteur et on introduit du dioxyde de carbone dans le réacteur jusqu'a une pression de 9,2 MPa. Durant cette opération d'introduction de dioxyde de carbone, la température est maintenue à 23°C. Le milieu est alors agité avec une vitesse de 210 t/min. On augmente alors graduellement la température du réacteur jusqu'à une valeur de 45°C, ce qui entraîne une augmentation de la pression à une valeur de 20 MPa. Dans ces conditions de température et de pression, le dioxyde de carbone se trouve à l'état supercritique et le Précirol^{®} ATO 5 est solubilisé dans le dioxyde de carbone. Le réacteur contient donc une dispersion des particules à enrober dans une solution de Précirol^{®} ATO 5. Après 1 heure d'agitation à 45°C et 20 MPa, on diminue graduellement et de manière contrôlée la température du réacteur jusqu'à une température de 17°C. Au cours de ce refroidissement, la pression diminue jusqu'à environ 6,5 MPa. La durée de l'étape de refroidissement est de 30 min. La diminution de température et de pression du milieu provoque une séparation de phase de la solution de Précirol^{®} ATO 5 dans le dioxyde de carbone. Le Précirol^{®} ATO 5 se dépose à la surface des particules à enrober et un phénomène de granulation se produit conduisant à l'obtention de microparticules contenant des particules protéiques dispersées dans la matrice de Précirol^{®} ATO 5. On diminue ensuite la pression du réacteur jusqu'à la pression atmosphérique par échappement contrôlé du dioxyde de carbone vers une ligne d'évent. La durée de cette étape de décompression est de 45 minutes.

Dès retour à pression atmosphérique, on ouvre le réacteur et on récupère 2,45 g de microparticules enrobées. Les microparticules sont ensuite tamisées à l'aide d'un tamis de maille 150 µm. Le rendement de tamisage est de 91%. Les microparticules tamisées, référencées M1 dans la suite, sont alors conditionnées dans des flacons en verre et sous atmosphère d'azote.

Les microparticules sont observées à l'aide d'un microscope optique muni d'une platine chauffante qui permet de porter les microparticules à une température d'environ 70°C, et de provoquer ainsi la fusion du Précirol^{®} ATO 5. Cette observation montre que les microparticules M1 sont constituées de particules protéiques dispersées dans le Précirol^{®} ATO 5 (figure 1).

La distribution granulométrique des microparticules M1, déterminée à l'aide d'un granulomètre laser équipé d'une cellule de mesure en voie liquide, est présentée dans le tableau 1. Le diamètre moyen des particules est de 101,5 µm.

**Tableau 1**

| Distribution granulométrique des microparticules M1 | |
|---|---|
| Microparticules M1 | |
| 10% | 35,24 µm |
| 20% | 57,4 µm |
| 50% | 97,8 µm |
| 80% | 142,01 µm |
| 90% | 171,15 µm |

Les microparticules M1 sont analysées par analyse enthalpique différentielle à balayage. Les microparticules M1 sont soumises à une augmentation de température de 10°C à 80°C à une vitesse de 10°C/min. Cette analyse thermique montre que le pic de fusion des microparticules M1 est de 57,9°C, avec une température de début de fusion ("onset") mesurée à 50,7°C. Aucun phénomène endothermique ou exothermique n'est mesuré pour des températures inférieures à 50,7°C.

Le taux de charge (TC) des microparticules M1 est mesuré par dosage immunoenzymatique (ELISA) à l'aide d'un kit spécifique de l'IFNα-2b humain (R&D Systems, référence 41100-1). Le taux de charge du lot de microparticules M1 est de 4.1% de lyophilisat protéique, soit un titre de 146 487 pg d'IFNα-2b par mg de microparticules.

### Exemple 2: Préparation des microparticules lipidiques M2 contenant l'interféron α-2b.

Un protocole de préparation identique à celui décrit dans l'exemple 1 est utilisé, à l'exception de la quantité de particules de lyophilisat protéique d'IFNα-2b à enrober, qui est de 77,24 mg pour 3 g de Précirol^{®} ATO 5 introduits dans le réacteur de IL.

Après enrobage des particules protéiques selon un mode opératoire identique à celui utilisé dans l'exemple 1, on récupère 2,42 g de microparticules enrobées. Ces microparticules sont tamisées à l'aide d'un tamis de 150 µm. Le rendement de tamisage est de 96,2%. Les microparticules tamisées, référencées M2 dans la suite, sont alors conditionnées dans des flacons en verre et sous atmosphère d'azote.

Les microparticules M2 sont observées à l'aide d'un microscope optique après fusion du Précirol^{®} ATO 5. On observe que les microparticules M2 sont constituées de particules de lyophilisat protéique dispersées dans le Précirol^{®} ATO 5.

La distribution granulométrique des microparticules M2, déterminée à l'aide d'un granulomètre laser équipé d'une cellule de mesure en voie liquide, est présentée dans le tableau 2. Le diamètre moyen des microparticules M2 est de 110,5 µm.

**Tableau 2**

| Distribution granulométrique des microparticules M2 | |
|---|---|
| Microparticules M2 | |
| 10% | 32,38 µm |
| 20% | 60,01 µm |
| 50% | 105,6 µm |
| 80% | 157,55 µm |
| 90% | 191,9 µm |

Les microparticules M2 sont analysées par analyse enthalpique différentielle à balayage. Les microparticules M2 sont soumises à une augmentation de température de 10°C à 80°C à une vitesse de 10°C/min. Cette analyse thermique montre que le pic de fusion des microparticules M2 est de 60°C, avec une température de début de fusion ("onset") mesurée à 48,3°C. Aucun phénomène endothermique ou exothermique n'est mesuré pour des températures inférieures à 48,3°C.

Le taux de charge des microparticules M2 est déterminé par dosage immunoenzymatique (ELISA) à l'aide d'un kit spécifique de l'IFNα-2b humain (R&D Systems). Le taux de charge (TC) du lot de microparticules M2 est de 1,6% de lyophilisat protéique, soit un titre de 45 893 pg d'IFNα-2b par mg de microparticules.

### Exemple 3: Utilisation des microparticules M1 pour améliorer la biodisponibilité de l'interféron α-2b (IFNα-2b) en injection sous-cutanée.

### 3.1. Mode opératoire.

### 3.1.1. Préparation des dispersions de microparticules d'interféron α-2b.

On utilise le lot de microparticules lipidiques M1 contenant l'interféron α-2b (IFNα-2b) enrobé par le Précirol^{®} ATO 5, obtenu en suivant le protocole opératoire décrit dans l'exemple 1.

On prépare deux solutions permettant de remettre en dispersion ces microparticules avant injection chez l'animal de la façon suivante:
- Solution de tensioactif: dans un bécher de 250 mL, on pèse précisément:
- 5g de Lutrol^{®}F68 de la société BASF,
- 5g de Solutol^{®}HS15 de la société BASF,
- 0,15g de Montanox^{®}20PHA de la société SEPPIC,
- 8,33g de D-Mannitol de la société CARLO ERBA. On ajoute 100 mL d'eau PPI (préparation pour injection) mesurés à l'aide d'une éprouvette. On maintient sous agitation d'un barreau aimanté jusqu'à complète solubilisation des constituants et jusqu'à obtenir une solution limpide. On effectue une filtration stérilisante de cette solution sur filtre 0,2µm PES (filtre seringue Nalgene).
- Solution de carboxyméthylcellulose (CMC): dans un bécher de 250 mL:
   - On verse précisément 100 mL d'eau PPI mesurés à l'aide d'une éprouvette,
   - On pèse précisément 1,25g de CMC (Blanose 7LF de la société Hercules),
   - On verse en pluie la CMC dans les 100 mL d'eau PPI sous agitation d'un barreau aimanté.

On maintient l'agitation jusqu'à complète dissolution. On effectue une filtration stérilisante de cette solution sur filtre 0,2µm PES (filtre seringue Nalgene). On effectue 7 pesées, de 263 mg +/- 1,5%, que l'on numérote de A à G, de microparticules M1, dans un flacon en verre type I d'un volume de 20 mL, muni d'un septum en bromobutyle pouvant être serti.

On calcule pour chaque flacon le volume précis X de solution de reconstitution totale qu'il faut ajouter pour obtenir une concentration finale en interféron α-2b de 3 793 333 pg/mL. On introduit dans le flacon contenant la pesée de microparticules un volume de solution de tensioactif égal à 60% du volume X précédemment calculé.

On ferme le flacon par un bouchon en bromobutyle, sans sertir, et on laisse sous agitation pendant 5 minutes à la vitesse de 22 tours/ minute. Après 5 minutes, on arrête l'agitation, on récupère le flacon et on ajoute un volume de solution de CMC égale à 40% du volume X précédemment calculé. On sertit le flacon et on le replace sous agitation pendant 5 minutes à la vitesse de 22 tours/minutes. Le flacon est ensuite placé sur agitateur Vortex Top-Mix 94323 commercialisé par la société Heidolph, pendant 2 secondes en agitation maximum et continue.

### 3.1.2. Préparation de la solution d'interféron α-2b non enrobé.

Une solution contenant l'interféron α-2b Gautier Cassara non enrobé est préparée de façon identique à la solution de redispersion: on effectue 1 pesée de 91,7 mg +/- 1,5%, d'interféron α-2b, dans un bécher de 100 mL, afin d'obtenir une concentration finale en interféron de 3 793 333 pg/mL. La solution obtenue est répartie dans 7 flacons en verre type I d'un volume de 20 mL, munis d'un septum en bromobutyle pouvant être serti.

### 3.1.3. Traitement des animaux et prélèvements.

Le nombre total de souris Swiss mâles, âgées de 8 à 10 semaines, ayant reçu une injection sous-cutanée de microparticules ou d'interféron alpha-2b non enrobé est de 70. Chaque souris est numérotée (codage universel de marquage aux oreilles) de 1 à 70, et 7 lots de 10 souris sont formés (lots A à G).

Chaque étude de formulation, microparticules ou interféron non enrobé, fait l'objet d'une analyse sur une semaine. Les injections débutent le matin à partir de 9 h. L'injection aux 10 souris constituant le lot A est réalisée en premier. L'injection aux 10 souris du lot B est réalisée à la suite, puis celle du lot C, et ainsi de suite jusqu'au lot G. L'heure exacte de début d'injection de chaque lot est notée, ce qui permet de corriger les résultats en fonction du temps exact, et non théorique, séparant l'injection du prélèvement pour chaque lot. L'aspiration de la suspension de microparticules dans les seringues est réalisée avec une aiguille de 20 Gauge (1 aiguille par lot) et les injections sont réalisées avec des aiguilles de 25 Gauge (aiguille changée pour chaque souris). Les solutions sont homogénéisées sur agitateur Vortex pendant 2 secondes entre chaque prélèvement.

| **Jour** | **Nombre d'heure après injection** | **Lot prélevé** |
|---|---|---|
| Mardi | 2 | A |
| " | 4 | B |
| " | 5 | C |
| Mercredi | 8 | D |
| " | 24 | E |
| " | 26 | F |
| " | 28 | G |
| " | 30 | A |
| " | 32 | B |
| Jeudi | 48 | C |
| " | 53 | D |
| " | 56 | E |
| Vendredi | 72 | F |
| " | 77 | G |
| Lundi | 144 | A |
| " | 149 | B |

Les 10 souris constituant un même lot sont numérotées, et les prélèvements sont réalisés dans le même ordre que les injections, ceci afin que le temps séparant injection et prélèvement soit le même pour toutes les souris d'un même lot. L'heure exacte de début de prélèvement de chaque lot est notée. Les lots de souris d'une même analyse font l'objet de prélèvements tour à tour au cours de la semaine, de façon à ce qu'aucun lot de souris ne subisse plus d'un prélèvement plus d'une fois par jour, selon le protocole suivant: les prélèvements ont été interrompus dès que les dosages ELISA faisaient apparaître une concentration en plus d'une fois par jour, selon le protocole suivant: les prélèvements ont été interrompus dès que les dosages ELISA faisaient apparaître une concentration en interféron α-2b circulant non significative (la limite de quantification pour le test ELISA est de 12,5 UI/mL).

Le volume de sang prélevé par souris au niveau du sinus retro-orbital est de 400 µL. Les échantillons sanguins sont centrifugés et les sérums sont prélevés et congelés immédiatement, dans l'attente des dosages ELISA. Le dosage de la concentration sérique en interféron α-2b de chaque échantillon recueilli est réalisé par technique ELISA (dosage immuno-enzymatique). Pour certains échantillons présentant une concentration d'interféron α-2b circulant dépassant la gamme de dosage ELISA, une dilution a été réalisée avec du tampon PBS, pH 7,4, 0,01% Montanox 20 DF, 0,02% acide de sodium.

### 3.2. Résultats.

L'ensemble des résultats sériques obtenus avec les microparticules M1, est résumé dans le tableau 3 ci-dessous:

**Tableau 3:**

| Concentration sérique en interféron α-2b humain en fonction du temps après l'injection d'une suspension de microparticules M1 à raison de 500 000 UI par souris. | | | |
|---|---|---|---|
| | | Concentration (pg/mL) | |
| Temps théorique | Temps réels (H) | Moyenne | Ecartype |
| 0 | 0,00 | 0 | 0 |
| 2 | 2,75 | 21051,76 | 6059,95 |
| 4 | 4,50 | 10571,51 | 2565,49 |
| 5 | 5,17 | 8141,79 | 1099,58 |
| 8 | 7,42 | 5701,16 | 1240,16 |
| 25 | 23,50 | 298,34 | 97,14 |
| 27 | 25,08 | 223,47 | 71,09 |
| 29 | 27,00 | 247,99 | 54,49 |
| 31 | 30,50 | 257,40 | 54,53 |
| 33 | 32,08 | 281,83 | 103,99 |
| 48 | 48,00 | 121,37 | 44,90 |
| 53 | 52,42 | 115,75 | 37,85 |
| 56 | 55,08 | 71,51 | 31,53 |
| 72 | 71,00 | 61,99 | 28,96 |
| 77 | 75,42 | 39,24 | 15,04 |
| 144 | 144,58 | 0,00 | 0,00 |
| 149 | 149,25 | 0,00 | 0,00 |

L'ensemble des résultats sériques obtenus avec l'interféron α-2b (IFNα-2b) Gautier Cassara (INF^{®}) non enrobé, est résumé dans le tableau 4 ci-dessous:

**Tableau 4**

| Concentration sérique en interféron α-2b humain en fonction du temps après l'injection d'une solution d'interféron α-2b (INF^{®}) non enrobé à raison de 500 000 UI par souris. | | | |
|---|---|---|---|
| | | Concentration (pg/mL) | |
| temps théorique | Temps en heures | Moyenne | Ecartype |
| 0 | 0,00 | 0 | 0 |
| 2 | 2,00 | 33737,17 | 5188,49 |
| 4 | 3,75 | 13749,07 | 3595,19 |
| 5 | 4,67 | 4118,72 | 830,51 |
| 8 | 7,33 | 482,33 | 115,71 |
| 25 | 23,33 | 149,19 | 32,23 |
| 27 | 25,08 | 100,44 | 18,73 |
| 29 | 26,92 | 125,74 | 34,78 |
| 31 | 30,00 | 83,13 | 25,04 |
| 33 | 31,67 | 77,90 | 20,24 |
| 48 | 47,67 | 19,00 | 12,66 |
| 53 | 52,42 | 33,33 | 7,54 |
| 56 | 55,25 | 15,43 | 5,25 |
| 72 | 71,42 | 7,19 | 5,50 |
| 77 | 75,92 | 6,34 | 4,37 |

Les concentrations en interféron alpha dosées au cours du temps dans le sérum des animaux ayant reçu soit de l'interféron α-2b non enrobé, soit des microparticules M1 sont comparées dans les figures 1A à 1E.

L'aire sous la courbe (AUC ou "area under the curve") représentatif de la biodisponibilité absolue de la protéine, calculée par modélisation en triangle et rectangle des aires obtenues entre chaque temps de prélèvements, est rassemblée dans les tableaux 5 et 6 suivants dans lesquels les concentrations sont exprimées en UI/mL et non en pg/mL. (1 UI=6,828 pg d'interféron α-2b Gautier Cassara).

**Tableau 5**

| Calcul de l'aire sous la courbe (AUC) : Interféron alpha-2b non enrobé (en (UI/mL) x h) | | | | |
|---|---|---|---|---|
| | | | | |
| temps | concentration | aire triangle | aire rectangle | aire totale |
| 0 | 0,000 | / | / | / |
| 2 | 4941,003 | 4941,0 | / | 4941,0 |
| 3,75 | 2013,630 | 2561,5 | 3523,9 | 6085,3 |
| 4,67 | 603,211 | 648,8 | 555,0 | 1203,7 |
| 7,33 | 70,641 | 708,3 | 187,9 | 896,2 |
| 23,33 | 21,850 | 390,3 | 349,6 | 739,9 |
| 25,08 | 14,711 | 6,2 | 25.7 | 32,0 |
| 26,92 | 18,415 | 3.4 | 27,1 | 30,5 |
| 30,00 | 12,174 | 9,6 | 37,5 | 47,1 |
| 31,67 | 11,408 | 0.6 | 19,1 | 19.7 |
| 47.67 | 2,783 | 69,0 | 44,5 | 113.5 |
| 52.42 | 4,882 | 5.0 | 13,2 | 18,2 |
| 55,25 | 2,260 | 3,7 | 6,4 | 10,1 |
| 71,42 | 1,053 | 9,8 | 17,0 | 26,8 |
| 75,92 | 0,93 | 0,3 | 4,2 | 4,5 |
| | | | | |
| | | | Total : | 14168,5 |

**Tableau 6**

| Calcul de l'aire sous la courbe : Microparticules M 1 | | | | |
|---|---|---|---|---|
| | | | | |
| temps | concentration | aire triangle | aire rectangle | aire totale |
| 0 | 0,000 | / | / | / |
| 2,75 | 3083,151 | 4239.3 | / | 4239,3 |
| 4,5 | 1548,259 | 1343,0 | 2709,5 | 4052,5 |
| 5,17 | 1192,413 | 119,2 | 798,9 | 918,1 |
| 7,42 | 834,968 | 402,1 | 1878,7 | 2280,8 |
| 23,50 | 43,693 | 6361,8 | 702,6 | 7064,4 |
| 25,08 | 32,729 | 8,7 | 51,7 | 60,4 |
| 27,00 | 36,319 | 3,4 | 62,8 | 66,3 |
| 30,50 | 37,698 | 2,4 | 127.1 | 129,5 |
| 32,08 | 41,276 | 2,8 | 59,6 | 62,4 |
| 48,00 | 17,775 | 187.1 | 283,0 | 470,1 |
| 52.42 | 16,952 | 1,8 | 74,9 | 76,7 |
| 55,08 | 10,474 | 8,6 | 27,9 | 36,5 |
| 71,00 | 9,080 | 11,1 | 144,6 | 155,6 |
| 75,42 | 5,740 | 7,4 | 25,4 | 32,8 |
| | | | | |
| | | | Total : | 19645,4 |

L'aire sous la courbe (AUC) obtenue pour les microparticules lipidiques M1 contenant le lyophilisat protéique est supérieure de 38,6% à l'AUC obtenue pour la protéine administrée seule (non enrobée) à la même dose.

### Exemple 4: Utilisation des microparticules M2 pour améliorer la biodisponibilité de l'interféron α-2b (IFNα-2b) en injection sous-cutanée.

### 4.1. Mode opératoire.

### 4.1.1. Préparation de la dispersion de microparticules.

On utilise le lot de microparticules lipidiques M2 contenant l'interféron α-2b (IFNα-2b) enrobé par le Précirol^{®} ATO 5, obtenu en suivant le protocole opératoire décrit dans l'exemple 2.

On prépare les deux solutions permettant de remettre en dispersion ces microparticules avant injection chez l'animal de façon identique à celle de l'exemple 3. On effectue 7 pesées, de 204 mg +/-2%, que l'on numérote de A à G, de microparticules M2, dans un flacon en verre type I d'un volume de 20mL muni d'un septum en bromobutyle, pouvant être serti. On calcule pour chaque flacon le volume précis X de solution de reconstitution totale qu'il faut ajouter pour obtenir une concentration finale en interféron de 948 333 pg/mL.

On introduit dans le flacon contenant la pesée des microparticules un volume de solution de tensioactif égal à 60% du volume X précédemment calculé.

On ferme le flacon par un bouchon en bromobutyle, sans sertir, et on laisse sous agitation pendant 5 minutes à la vitesse de 22 tours/minute. Après 5 minutes, on arrête l'agitation, on récupère le flacon et on ajoute un volume de solution de CMC égale à 40% du volume X précédemment calculé. On sertit le flacon et on le remplace sous agitation pendant 5 min à la vitesse de 22 t/min. Le flacon est ensuite placé sur agitateur Vortex Top-Mix 94323 commercialisé par la société Heidolph, pendant 2 secondes en agitation maximum et continue.

### 4.1.2. Préparation de la solution d'interféron α-2b non enrobé.

Une solution contenant l'interféron α-2b Gautier Cassara non enrobé est préparée de façon identique: on effectue 1 pesée de 22,9 mg +/- 1,5%, d'interféron α-2b, dans un bécher de 100 mL, afin d'obtenir une concentration finale en interféron alpha-2b de 948 333 pg/mL. La solution obtenue est répartie dans 7 flacons en verre type I d'un volume de 20 mL, munis d'un septum bromobutyle pouvant être serti.

### 4.1.3. Traitement des animaux et prélèvements.

Le nombre total de souris Swiss mâles âgées de 8 à 10 semaines, ayant reçu une injection sous-cutanée de microparticules ou d'interféron α-2b non enrobé est de 70. Chaque souris est numérotée (codage universel de marquage aux oreilles) de 1 à 70, et 7 lots de 10 souris sont formés (lots A à G).

Chaque étude de formulation, microparticules ou interféron α-2b non enrobé, fait l'objet d'une analyse sur une semaine. Les injections et les prélèvements sont réalisés en suivant un protocole identique à celui de l'exemple 3.

Les lots de souris d'une même analyse font l'objet de prélèvements tour à tour au cours de la semaine, de façon à ce qu'aucun lot de souris ne subisse plus d'un prélèvement plus d'une fois par jour, selon le protocole suivant:

| **Jour** | **Nombre d'heure après injection** | **Lot prélevé** |
|---|---|---|
| Lundi | 2 | A |
| " | 4 | B |
| " | 5 | C |
| " | 8 | D |
| Mardi | 24 | E |
| " | 26 | F |
| " | 28 | G |
| " | 30 | A |
| " | 32 | B |
| Mercredi | 48 | C |
| " | 53 | D |
| " | 56 | E |
| Jeudi | 72 | F |
| " | 77 | G |
| Vendredi | 96 | A |
| " | 101 | B |

Les prélèvements ont été interrompus dès que les dosages ELISA faisaient apparaître une concentration en interféron alpha circulant non significative (la limite de quantification pour ce test ELISA est de 12,5 UI/mL). Le volume de sang prélevé par souris au niveau du sinus retro-orbital est de 400 µL. Les échantillons sanguins sont centrifugés et les sérums sont prélevés et congelés immédiatement, dans l'attente des dosages ELISA.

Le dosage de la concentration sérique en interféron α-2b humain de chaque échantillon recueilli est réalisé par technique ELISA (dosage immuno-enzymatique). Pour certains échantillons présentant une concentration d'interféron α-2b circulant dépassant la gamme de dosage ELISA, une dilution a été réalisée avec du tampon PBS, pH 7,4, 0,01% Montanox 20 DF, 0,02% acide de sodium.

### 4.2. Résultats.

L'ensemble des résultats sériques obtenus avec les microparticules M2, est résumé dans le tableau 7 ci-dessous:

**Tableau 7**

| Concentration sérique en interféron α-2b humain des animaux en fonction du temps après l'injection d'une suspension de microparticules M2 à raison de 125 000 UI par souris. | | | |
|---|---|---|---|
| | | Concentration (pg/mL) | |
| temps théorique | Temps en heures | Moyenne | Ecartype |
| 0 | 0,00 | 0 | 0 |
| 2 | 2,58 | 10194,39 | 3921,13 |
| 4 | 4,00 | 8183,94 | 2930,55 |
| 5 | 4,92 | 5557,55 | 3788,00 |
| 8 | 7,50 | 3606,26 | 1596,34 |
| 25 | 23,08 | 271,62 | 84,12 |
| 27 | 24,92 | 324,22 | 149,05 |
| 29 | 26,75 | 247,36 | 109,86 |
| 31 | 30,25 | 245,73 | 41,64 |
| 33 | 31,92 | 243,42 | 52,81 |
| 48 | 48,17 | 90,57 | 41,18 |
| 53 | 52,58 | 129,82 | 59,76 |
| 56 | 54,42 | 98,86 | 56,60 |
| 72 | 70,92 | 9,82 | 9,98 |
| 77 | 75,67 | 4,22 | 7,88 |
| 96 | 96,25 | 0,00 | 0,00 |
| 101 | 100,75 | 0,00 | 0,00 |

L'ensemble des résultats sériques obtenus avec l'interféron α-2b non enrobé, est résumé dans le tableau 8 ci-dessous.

**Tableau 8**

| Concentration sérique en interféron α-2b humain des animaux en fonction du temps après l'injection d'une solution d'interféron alpha non enrobé à raison de 125 000 UI par souris. | | | |
|---|---|---|---|
| | | Concentration (pg / m L) | |
| temps théorique | Temps en heures | Moyenne | Ecartype |
| 0 | 0 | 0,00 | 0,00 |
| 2 | 1,83 | 8812,16 | 2776,03 |
| 4 | 4,08 | 1022,84 | 618,94 |
| 5 | 4,92 | 548,68 | 266,12 |
| 8 | 7,33 | 15,89 | 21,03 |
| 25 | 23,42 | 9,17 | 13,70 |
| 27 | 25 | 9,40 | 13,47 |
| 29 | 27,25 | 0,75 | 1,25 |
| 31 | 30 | 5,76 | 7,31 |
| 33 | 31,66 | 0,00 | 0,00 |
| 48 | 47,5 | 0,00 | 0,00 |
| 53 | 52,33 | 0,00 | 0,00 |
| 56 | 55,17 | 0,00 | 0,00 |
| 72 | 71 | 0,00 | 0,00 |

Les concentrations en interféron α dosées au cours du temps dans le sérum des animaux ayant reçu soit de l'interféron α-2b, soit des microparticules M2 sont comparées dans les figures 2A et 2B.

L'aire sous la courbe est calculée par la méthode décrite dans l'exemple 3 et rassemblée dans les tableaux 9 et 10 suivants:

**Tableau 9**

| Calcul de l'aire sous la courbe (AUC) : Interferon alpha-2b non enrobé (en (UI/mL) x h) | | | | |
|---|---|---|---|---|
| | | | | |
| temps | concentration | aire triangle | aire rectangle | aire totale |
| 0 | 0,0 | / | / | / |
| 1,83 | 1290,6 | 1180,9 | / | 1180,9 |
| 4,08 | 149,8 | 1283,4 | 337,1 | 1620,4 |
| 4,92 | 80,4 | 29,2 | 67,5 | 96,7 |
| 7,33 | 2,3 | 94,0 | 5,6 | 99,6 |
| 23,42 | 1,3 | 7,9 | 21,6 | 29,5 |
| 25 | 1,4 | 0,0 | 2,1 | 2,1 |
| 27,25 | 0,1 | 1,4 | 0,2 | 1,7 |
| 30 | 0,8 | 1,0 | 0,3 | 1,3 |
| 31,66 | 0,0 | 0,7 | 0,0 | 0,7 |
| 47,5 | 0,0 | 0,0 | 0,0 | 0,0 |
| 52,33 | 0,0 | 0,0 | 0,0 | 0,0 |
| 55,17 | 0,0 | 0,0 | 0,0 | 0,0 |
| 71 | 0,0 | 0,0 | 0,0 | 0,0 |
| | | | | |
| | | | Total: | 3033,0 |

**Tableau 10**

| Calcul de l'aire sous la courbe (AUC) : Microparticules M2 (en (UI/mL) x h) | | | | |
|---|---|---|---|---|
| | | | | |
| temps | concentration | aire triangle | aire rectangle | aire totale |
| 0,00 | 0,00 | / | #VALEUR! | / |
| 2,58 | 1493,03 | 1926,0 | 0,0 | 1926,0 |
| 4,00 | 1198,59 | 209,1 | 1702,0 | 1911,0 |
| 4,92 | 813,94 | 176.9 | 748,8 | 925,8 |
| 7,50 | 528,16 | 368,7 | 1362.6 | 1731.3 |
| 23,08 | 39,78 | 3804,5 | 619,8 | 4424,2 |
| 24,92 | 47.48 | 7,1 | 73,2 | 80,3 |
| 26,75 | 36,23 | 10,3 | 66,3 | 76,6 |
| 30,25 | 35,99 | 0,4 | 126,0 | 126,4 |
| 31,92 | 35.65 | 0,3 | 59,5 | 59,8 |
| 48,17 | 13,26 | 181,9 | 215,5 | 397,4 |
| 52,58 | 19,01 | 12,7 | 58,5 | 71,2 |
| 54,42 | 14,48 | 4,2 | 26,6 | 30,8 |
| 70,92 | 1,44 | 107,6 | 23,7 | 131,3 |
| 75,67 | 0,62 | 1.9 | 2,9 | 4,9 |
| 96,25 | 0,00 | 6,4 | 0,0 | 6,4 |
| 100,75 | 0,00 | 0,0 | 0,0 | 0,0 |
| | | | | |
| | | | Total : | 11903,4 |

L'aire sous la courbe (AUC) obtenue pour les microparticules lipidiques M2 contenant le lyophilisat protéique est multipliée par un facteur 3,9 par rapport à l'AUC obtenue pour la protéine administrée seule (non enrobée) à la même dose.

### Exemple 5: Utilisation des microparticules lipidiques M2 avec une solution de reconstitution contenant du Lutrol^{®} F127, pour améliorer la biodisponibilité de l'interféron α-2b (IFNα-2b) en injection sous-cutanée.

### 5.1. Mode opératoire.

### 5.1.1. Préparation de la dispersion de microparticules.

On utilise le même lot de microparticules lipidiques M2 d'interféron α-2b (IFNα-2b) enrobé par le PRÉCIROL^{®} ATO 5 que celui de l'exemple 4, dont le taux de charge TC est 1,6% en lyophilisat de protéine.

On prépare une solution permettant de remettre en dispersion ces microparticules avant injection chez l'animal de la façon suivante: dans un bécher de 250 mL, on pèse précisément:
- 3g de Lutrol^{®} F68 de la société BASF,
- 3g de Solutol^{®} HS15 de la société BASF,
- 0,09g de Montanox^{®} 20PHA de la société SEPPIC,
- 5,1g de D-Mannitol de la société CARLO ERBA.

On ajoute 100 mL d'eau PPI mesurés à l'aide d'une éprouvette, et on maintient sous agitation d'un barreau aimanté jusqu'à complète solubilisation des constituants et jusqu'à obtenir une solution limpide. On place le bécher dans un bain-marie d'eau glacée. On pèse 7,5 g de Lutrol^{®} F127 de la société BASF et on l'incorpore en pluie dans la solution précédente. On maintient l'agitation jusqu'à dissolution complète. On effectue une filtration stérilisante de cette solution sur filtre 0,2µm PES (filtre seringue Nalgene).

On effectue 7 pesées, de 204 mg +/-2%, que l'on numérote de A à G, de microparticules M2, dans un flacon en verre type I d'un volume de 20 mL muni d'un septum en bromobutyle pouvant être serti. On calcule pour chaque flacon le volume précis X de solution de reconstitution totale qu'il faut ajouter pour obtenir une concentration finale en interféron de 948 333 pg/mL. On introduit dans le flacon contenant la pesée de microparticules M2 ce volume de solution. On sertit le flacon par un bouchon en bromobutyle, et on laisse sous agitation pendant 10 min à la vitesse de 22 t/min. Le flacon est ensuite placé sur agitateur Vortex Top-Mix 94323 commercialisé par la société Heidolph, pendant 2 secondes en agitation maximum et continue.

Les résultats obtenus avec l'interféron α-2b non enrobé tel que décrit dans l'exemple 4 sont utilisés à titre de comparaison.

### 5.1.2. Traitement des animaux et prélèvements.

Le nombre total de souris Swiss mâles âgées de 8 à 10 semaines, ayant reçu une injection sous-cutanée de microparticules ou d'interféron alpha-2b non enrobé est de 70. Chaque souris est numérotée (codage universel de marquage aux oreilles) de 1 à 70, et 7 lots de 10 souris sont formés (lots A à G).

Chaque étude de formulation, microparticules ou interféron non enrobé, fait l'objet d'une analyse sur une semaine. Les injections et les prélèvements sont réalisés en suivant un protocole identique à celui de l'exemple 4. Les lots de souris d'une même analyse font l'objet de prélèvements tour à tour au cours de la semaine, de façon à ce qu'aucun lot de souris ne subisse plus d'un prélèvement plus d'une fois par jour, selon le protocole suivant:

| **Jour** | **Nombre d'heure après injection** | **Lot prélevé** |
|---|---|---|
| Lundi | 2 | A |
| " | 4 | B |
| " | 5 | C |
| " | 8 | D |
| Mardi | 24 | E |
| " | 26 | F |
| " | 28 | G |
| " | 30 | A |
| " | 32 | B |
| Mercredi | 48 | C |
| " | 53 | D |
| " | 56 | E |
| Jeudi | 72 | F |
| " | 77 | G |
| Vendredi | 96 | A |
| " | 101 | B |

Les prélèvements ont été interrompus dès que les dosages ELISA faisaient apparaître une concentration en interféron α-2b circulant non significative (la limite de quantification pour ce test ELISA est de 12,5 UI/mL). Le volume de sang prélevé par souris au niveau du sinus retro-orbital est de 400 µL. Les échantillons sanguins sont centrifugés et les sérums sont prélevés et congelés immédiatement, dans l'attente des dosages ELISA.

La concentration sérique en interféron alpha-2b humain de chaque échantillon recueilli est mesurée par technique ELISA (dosage immuno-enzymatique). Pour certains échantillons présentant une concentration d'interféron α-2b circulant dépassant la gamme de dosage ELISA, une dilution a été réalisée avec du tampon PBS, pH 7,4, 0,01% Montanox 20 DF, 0,02% acide de sodium.

### 5.2. Résultats.

L'ensemble des résultats sériques obtenus avec les microparticules M2 dans la solution de reconstitution à base de Lutrol^{®} F127, est résumé dans le tableau 11 ci-dessous.

**Tableau 11**

| Concentration sérique en interféron α-2b humain en fonction du temps après l'injection d'une suspension de microparticules M2 à raison de 125 000 UI par souris. | | | |
|---|---|---|---|
| | | Concentration (pg/mL) | |
| temps théorique | Temps en heures | Moyenne | Ecartype |
| 0 | 0,00 | 0 | 0 |
| 2 | 2,17 | 3945,54 | 1347,67 |
| 4 | 3,92 | 6366,02 | 2613,22 |
| 5 | 4,50 | 3044,54 | 1085,99 |
| 8 | 7,08 | 3202,59 | 1087,07 |
| 25 | 23,58 | 207,89 | 90,07 |
| 27 | 24,75 | 218,50 | 66,70 |
| 29 | 26,58 | 259,17 | 96,96 |
| 31 | 30,00 | 289,06 | 124,85 |
| 33 | 31,58 | 384,13 | 130,94 |
| 48 | 47,67 | 65,37 | 46,70 |
| 53 | 52,17 | 46,15 | 51,63 |
| 56 | 54,75 | 28,74 | 32,74 |
| 72 | 70,92 | 0,00 | 0,00 |
| 77 | 75,42 | 1,92 | 5,76 |
| 96 | 96,25 | 3,42 | 7,95 |

Les concentrations en interféron α dosées au cours du temps dans le sérum des animaux ayant reçu soit de l'interféron α-2b non enrobé, soit des microparticules M2 reprises dans la solution de reconstitution à base de Lutrol^{®} F127 sont comparées dans les figures 3A et 3B.

L'aire sous la courbe est calculée par la méthode décrite dans l'exemple 3 et est illustrée dans les tableaux 12 et 13 suivants dans lesquels Les concentrations sont exprimées en UI/mL et non en pg/mL. Rappel 1 UI=6,828 pg d'interféron α-2b Gautier Cassara.

**Tableau 12**

| Calcul de l'aire sous la courbe (AUC) : Interferon alpha-2b non enrobé (en (UI/mL) x h) | | | | |
|---|---|---|---|---|
| | | | | |
| temps | concentration | aire triangle | aire rectangle | aire totale |
| 0 | 0,0 | / | / | / |
| 1,83 | 1290,6 | 1180,9 | / | 1180,9 |
| 4,08 | 149,8 | 1283,4 | 337,1 | 1620,4 |
| 4,92 | 80,4 | 29,2 | 67,5 | 96,7 |
| 7,33 | 2,3 | 94,0 | 5,6 | 99,6 |
| 23,42 | 1,3 | 7,9 | 21,6 | 29,5 |
| 25 | 1,4 | 0,0 | 2,1 | 2,1 |
| 27,25 | 0,1 | 1,4 | 0,2 | 1,7 |
| 30 | 0,8 | 1,0 | 0,3 | 1,3 |
| 31,66 | 0,0 | 0,7 | 0,0 | 0,7 |
| 47,5 | 0,0 | 0,0 | 0,0 | 0,0 |
| 52,33 | 0,0 | 0,0 | 0,0 | 0,0 |
| 55,17 | 0,0 | 0,0 | 0,0 | 0,0 |
| 71 | 0,0 | 0,0 | 0,0 | 0,0 |
| | | | | |
| | | | Total : | 3033,0 |

**Tableau 13**

| Calcul de l'aire sous la courbe Microparticules M2 ; nouvelle solution de remise en suspension (en (UI/mL) x h) | | | | |
|---|---|---|---|---|
| | | | | |
| temps | concentration | aire triangle | aire rectangle | aire totale |
| 0 | 0,0 | / | / | / |
| 2,17 | 577,8 | 627,0 | / | 627,0 |
| 3,92 | 932,3 | 310,2 | 1011,2 | 1321,4 |
| 4,50 | 445,9 | 141,1 | 258,6 | 399,7 |
| 7,08 | 469,0 | 29,9 | 1150,4 | 1180,3 |
| 23,58 | 30,4 | 3618,4 | 502,4 | 4120,8 |
| 24,75 | 32,0 | 0,9 | 35,6 | 36,5 |
| 26,58 | 38,0 | 5,5 | 58,6 | 64,0 |
| 30,00 | 42,3 | 7,5 | 129,8 | 137,3 |
| 31,58 | 56,3 | 11,0 | 66,9 | 77,9 |
| 47,67 | 9,6 | 375,6 | 154,0 | 529,6 |
| 52,17 | 6,8 | 6,3 | 30,4 | 36,7 |
| 54,75 | 4,2 | 3,3 | 10,9 | 14,1 |
| 70,92 | 0,0 | 34,0 | 0,0 | 34,0 |
| 75,42 | 0,3 | 0,6 | 0,0 | 0,6 |
| 96,25 | 0,5 | 2,3 | 5,9 | 8,2 |
| | | | | |
| | | | Total : | 8588,1 |

L'aire sous la courbe (AUC) obtenue pour les microparticules lipidiques M2 contenant le lyophilisat protéique, redispersées dans une solution de Lutrol^{®} F127, est multipliée par un facteur 2,83 par rapport à l'AUC obtenue pour la protéine administrée seule (non enrobée) à la même dose.

Le tableau 14 récapitule les valeurs du paramètre AUC, représentatif de la biodisponibilité absolue de la protéine, trouvées dans les exemples 3, 4 et 5.

**Tableau 14**

| **Formulation** | **TC(%)** | **Dose (UI/souris)** | **AUC (UI/mL) x h** |
|---|---|---|---|
| Interféron alpha 2-b en solution (référence) | / | 500 000 | 14169 |
| Microparticules M1 redispersées dans solution de CMC | 4,1 | 500 000 | 19645 |
| Interféron alpha 2-b en solution (référence) | / | 125 000 | 3033 |
| Microparticules M2 redispersées dans solution de CMC | 1,6 | 125 000 | 11903 |
| Microparticules M2 redispersées dans solution de Lutrol F127 | 1,6 | 125 000 | 8588 |

## Revendications

1. Utilisation du distéarate de glycérol comme agent de formulation pour augmenter la biodisponibilité de principes actifs protéiques dans des formulations injectables sous-cutanées ou intramusculaires, ledit principe actif étant l'interféron α-2b.

2. Utilisation selon la revendication 1, **caractérisée en ce que** les principes actifs protéiques sont contenus dans des particules ayant une taille comprise entre 0,1 et 100 µm, de préférence entre 1 et 25 µm.

3. Utilisation selon l'une quelconque des revendications 1 à 2, **caractérisée en ce que** le principe actif protéique est formulé avec des lipides sous forme de nanocapsule, microcapsule (structure coeur protéique-écorce lipidique), ou nanosphère, microsphère, implants cylindriques ou discoïdes (structure matricielle lipidique contenant le principe actif protéique dispersé), de préférence sous forme d'une structure matricielle.

4. Utilisation selon la revendication 1, **caractérisée en ce que** les systèmes formulés ont une taille comprise entre 1 et 500 µm.

5. Utilisation selon la revendication 4, **caractérisée en ce que** les particules de principe actif protéique sont formulées dans un système particulaire lipidique ayant un diamètre compris entre 10 et 100 µm.

6. Utilisation selon la revendication 4, **caractérisée en ce que** les particules de principe actif protéique sont formulées dans un implant lipidique dont le diamètre est compris entre 10 et 300 µm.

7. Utilisation selon l'une quelconque des revendications 1 à 6, **caractérisée en ce que** les particules de principe actif protéique ont une teneur en principe actif protéique entre 0,01 et 100%.

8. Utilisation selon l'une quelconque des revendications 1 à 7, **caractérisée en ce que** la teneur des systèmes formulés en particules de principe actif protéique est comprise entre 0,1 % et 95%, avantageusement entre 0,5 et 25%, encore plus avantageusement entre 1 et 10%.

9. Utilisation selon l'une quelconque des revendications 1 à 8, **caractérisée en ce que** les microsphères matricielles sont obtenues par encapsulation du principe actif protéique en utilisant un fluide à pression supercritique.

## Patentansprüche

1. Verwendung von Glyceroldipalmitostearat als Formulierungsmittel zur Erhöhung der Bioverfügbarkeit von Proteinwirkstoffen in subkutanen oder intramuskulären Injektionsformulierungen, wobei der Wirkstoff das Interferon α-2b ist.

2. Verwendung nach Anspruch 1, **dadurch gekennzeichnet, dass** die Proteinwirkstoffe in Partikeln enthalten sind, die eine Größe von zwischen 0,1 und 100 µm, bevorzugt zwischen 1 und 25 µm, haben.

3. Verwendung nach einem der vorstehenden Ansprüche 1 bis 2, **dadurch gekennzeichnet, dass** der Proteinwirkstoff mit Lipiden in Form von Nanokapseln, Mikrokapseln (Struktur Proteinkern-Lipidschale), oder Nanosphären, Mikrosphären, stäbchen- oder scheibenförmigen Implantaten (Lipid-Matrixstruktur, die den gestreuten Proteinwirkstoff enthält), bevorzugt in Form einer Matrixstruktur formuliert ist.

4. Verwendung nach Anspruch 1, **dadurch gekennzeichnet, dass** die formulierten Systeme eine Größe von zwischen 1 und 500 µm haben.

5. Verwendung nach Anspruch 4, **dadurch gekennzeichnet, dass** die Proteinwirkstoffpartikel in einem teilchenförmigen Lipidsystem formuliert sind, das einen Durchmesser von zwischen 10 und 100 µm hat.

6. Verwendung nach Anspruch 4, **dadurch gekennzeichnet, dass** die Proteinwirkstoffpartikel in einem Lipidimplantat formuliert sind, das einen Durchmesser von zwischen 10 und 300 µm hat.

7. Verwendung nach einem der vorstehenden Ansprüche 1 bis 6, **dadurch gekennzeichnet, dass** die Proteinwirkstoffpartikel einen Proteinwirkstoffgehalt von zwischen 0,01 und 100 % haben.

8. Verwendung nach einem der vorstehenden Ansprüche 1 bis 7, **dadurch gekennzeichnet, dass** der Gehalt der in Proteinwirkstoffpartikeln formulierten Systeme bei zwischen 0,1 % und 95 %, vorteilhaft bei zwischen 0,5 und 25 %, noch vorteilhafter bei zwischen 1 und 10 % liegt.

9. Verwendung nach einem der vorstehenden Ansprüche 1 bis 8, **dadurch gekennzeichnet, dass** die Matrixmikrosphären durch Einkapselung des Proteinwirkstoffs unter Verwendung eines auf überkritischem Druck befindlichen Fluids erzielt werden.

## Claims

1. The use of glycerol distearate as a formulating agent to increase the bioavailability of protein active ingredients in subcutaneous or intramuscular injectable formulations, said active ingredient being interferon α-2b.

2. The use according to claim 1, **characterized in that** the protein active ingredients are contained in particles ranging between 0.1 and 100 µm in size, preferably between 1 and 25 µm in size.

3. The use according to any one of claims 1 to 2, **characterized in that** the protein active ingredient is formulated with lipids in the form of a nanocapsule, microcapsule (lipid-core, protein-shell structure), or nanosphere, microsphere, cylindrical or discoidal implants (lipid matrix structure containing the dispersed protein active ingredient), preferentially in the form of a matrix structure.

4. The use according to claim 1, **characterized in that** the formulated systems have a size in the range between 1 and 500 µm.

5. The use according to claim 4, **characterized in that** the particles of protein active ingredient are formulated in a lipid particulate system having a diameter in the range between 10 and 100 µm.

6. The use according to claim 4, **characterized in that** the particles of protein active ingredient are formulated in a lipid implant of 10 to 300 µm in diameter.

7. The use according to any one of claims 1 to 6, **characterized in that** the particles of protein active ingredient have a protein active ingredient content between 0.01 and 100 %.

8. The use according to any one of claims 1 to 7, **characterized in that** the content of the formulated systems of particles of protein active ingredient is in the range between 0.1 % and 95 %, advantageously between 0.5 and 25 %, still more advantageously between 1 and 10 %.

9. The use according to any one of claims 1 to 8, **characterized in that** the matrix microspheres are obtained by encapsulation of the protein active ingredient by using a fluid at supercritical pressure.
